# EUROPÄISCHE PATENTSCHRIFT

(11) **EP 1 449 553 B1**
(45) Veröffentlichungstag und Bekanntmachung des Hinweises auf die Patenterteilung: **11.04.2007**
(21) Anmeldenummer: 04003236.9
(22) Anmeldetag: 13.02.2004
(51) Int. Cl.: A61M 5/165

(54) **Filter für medizinische und Laborzwecke, insbesondere für Blutanalysen**
Filter for medical and laboratory purposes, in particular for blood analysis
Filtre pour usage médical et pour laboratoire, en particulier pour analyse de sang

(30) Priorität: 21.02.2003 DE 20302819 U
(43) Veröffentlichungstag der Anmeldung: 25.08.2004
(73) Patentinhaber: Filtertek S.A., F-60128 Plailly (FR)
(72) Erfinder: Chiga, Antonio, 77230 Dammartin en Groele (FR)
(74) Vertreter: Brose, D. Karl

(56) Entgegenhaltungen:
- EP-A- 1 271 028
- WO-A-01/48141
- US-A- 4 319 996

## Beschreibung

Die Erfindung betrifft einen Filter für medizinische und Laborzwecke, insbesondere für Blutanalysen und dergleichen, mit einem zweiteiligen, aus einem Unterteil und einem Deckel bestehenden Gehäuse aus Kunststoff, wobei der Deckel einen Einlass und der Unterteil einen Auslass aufweist, und wobei zwischen Deckel und Unterteil eine auswechselbare Filtermembrane eingeklemmt ist und eine den Deckel mit dem Unterteil mediendicht verbindende Verbindungseinrichtung vorgesehen ist.

Derartige Filter sind allgemein bekannt und bestehen üblicherweise aus einem hülsenförmigen Körper als Unterteil des Gehäuses, wobei der Deckel mittels eines Gewindes im Drehverschluss mit dem hülsenförmigen Unterteil verbindbar ist. Derartige Filter werden in der Medizintechnik als Laborfilter oder für Blutanalysen verwendet, wobei spezielle Filtermembranen für die unterschiedlichsten Untersuchungen verwendet werden, welche häufig und schnell ausgewechselt werden müssen. Da derartige Filter, was ihre Gehäuse betrifft, etwa einen Durchmesser von 30 mm aufweisen, ist die Gewindeverbindung zwischen Deckel und Gehäuseunterteil ausgesprochen unhandlich, wobei hinzu kommt, dass sich beim Zuschrauben die Filtermembran verziehen kann. Darüber hinaus ist die Herstellung des Gehäuses für derartige Filter vergleichsweise schwierig, da bei den verwendeten Spritzgiesswerkzeugen spezielle drehbare Formkerne verwendet werden müssen, die es ermöglichen, eines der beiden Gewindeteile, d.h. das Innengewinde, zu entformen. Die Kosten der hierzu herzustellenden Werkzeuge sind vergleichsweise hoch.

Ein Dokument, das die Merkmale des Oberbegriffs des Anspruchs 1 offenbart, ist das WO 01/48141.

Der Erfindung liegt die Aufgabe zugrunde, einen Filter der oben angegebenen Art dahingehend zu verbessern, dass er die dem Stand der Technik anhaftenden Nachteile vermeidet und darüber hinaus wesentlich kostengünstiger herstellbar ist.

Bei einem Filter der eingangs genannten Art wird diese Aufgabe durch die Merkmale des Anspruchs 1 gelöst. Es ist offensichtlich, dass durch diese Merkmale das Gehäuse des Filters durch einfaches Zusammenpressen zusammengebaut werden kann, so dass die Filtermembran weder verzogen noch beschädigt werden kann. Darüber hinaus sind die zur Herstellung der beiden Gehäuseteile benötigten Werkzeuge insbesondere beim Spritzgiessen wesentlich kostengünstiger herstellbar.

Insbesondere ist es vorgesehen, dass die hakenförmigen Vorsprünge den Unterrand des Unterteils lösbar übergreifen. Hierdurch wird die Konstruktion vereinfacht, da keine besonderen komplementären Flächen für den Eingriff der hakenförmigen Vorsprünge vorgesehen werden müssen.

Es ist ferner vorgesehen, dass die Federhebel oberhalb ihres Drehpunkts nach oben vorstehende Betätigungslaschen aufweisen. Hierdurch lässt sich das Filtergehäuse wieder leicht öffnen, um ein Auswechseln der Filtermembran zu ermöglichen.

Die Federhebel sind bevorzugt einstückig mit dem aus Kunststoff bestehenden Deckel ausgebildet, wobei bei einer besonders bevorzugten Ausführungsform die Federhebel an seitlich vom Deckel vorstehenden bogenförmigen Laschen angeformt sind, welche den Drehpunkt des Federhebels bilden. Unter Ausnutzung der elastischen Eigenschaften des verwendeten Kunststoffs wird hierdurch gleichzeitig die Federkraft erzeugt und die Konstruktion erheblich vereinfacht.

Bevorzugt ist es, drei Federhebel um den Umfang des Deckels in gleichen Abständen verteilt vorzusehen, da hierdurch ein sicheres Anpressen des Deckels an dem Gehäuseunterteil gewährleistet ist.

Im einzelnen ist es ferner bevorzugt, dass zwischen dem Deckel und dem Unterteil eine zusammendrückbare Ringdichtung vorgesehen ist.

Die Ringdichtung ist bevorzugt in einem ringförmigen Kanal in der Unterseite des Deckels angeordnet bzw. befestigt.

Im einzelnen kann die Erfindung dadurch weitergebildet werden, dass der Kanal radial ausserhalb der Ringdichtung einen freien Ringraum aufweist, in welchen ein ringförmiger Vorsprung auf der Oberseite des Unterteils eingreift, welcher eine Auflagefläche der Ringdichtung umgibt. Hierdurch wird der Vorteil erzielt, dass beim Zusammenbau des Filtergehäuses automatisch der Deckel gegenüber dem Unterteil zentriert wird.

Bei einer besonders bevorzugten Ausführungsform ist vorgesehen, dass die Filtermembran in ihrem Durchmesser dem Innendurchmesser des ringförmigen Vorsprungs entspricht, derart, dass bei geschlossenem Gehäuse der Randbereich der Filtermembran zwischen der Ringdichtung und der Auflagefläche eingeklemmt ist. Hierdurch ist eine sichere und straffe Halterung der Filtermembran erreicht.

Im einzelnen ist es von Vorteil, dass in einer topfartigen Vertiefung des Unterteils innerhalb der Auflagefläche ein scheibenförmiger Stützkörper für die Filtermembran vorgesehen ist, wobei bei einer besonders bevorzugten Ausführungsform der Boden der Vertiefung mit sternförmig, den zentralen Auslass umgebenden Stützrippen versehen ist.

Im einzelnen ist es von Vorteil, dass der Stützkörper aus porösem Werkstoff besteht.

Durch diese Merkmale wird erreicht, dass eine Durchbiegung der Filtermembran beim Filtern vermieden wird.

Bei einer besonders bevorzugten Ausführungsform nach der Erfindung ist die Filtermembran mit einer seitlichen, aus dem geschlossenen Gehäuse vorstehenden Zunge versehen. Hierdurch wird erreicht, dass die Filtermembran beim Wechsel leicht eingelegt und befestigt werden kann, wobei die aus dem Gehäuse herausragende Zunge gleichzeitig zur Kennzeichnung der zur Zeit im Filtergehäuse befindlichen Membran dienen kann. Darüber hinaus wird auch eine einfache Entnahme beim Wechsel der Membran für die darauffolgende Anwendung erreicht. Weiterer Vorteil dieser Merkmale besteht darin, dass aufgrund dieser Kennzeichnung eine schnelle visuelle Zuordnung des Filtergehäuses während der Anwendung für Dritte möglich ist.

Bei einer Weiterbildung des Filters nach der Erfindung ist vorgesehen, dass radial ausserhalb des ringförmigen Vorsprungs an dem Unterteil ein weiterer Ringkanal vorgesehen ist, dessen Aussenwandung an der äussersten Unterkante von den hakenförmigen Vorsprüngen übergriffen wird. Hierdurch wird der Vorteil erzielt, dass der die Filtermembran tragende Bereich des Gehäuses nicht von den durch die Federhebel ausgeübten Klemmkräfte beeinflusst wird. Darüber hinaus kann der weitere Ringkanal in vorteilhafter Weise beim Wechsel der Filtermembran zurückgebliebenes Filtrat oder zu filtrierende Flüssigkeit auffangen.

Diese Ausführungsform kann dadurch weitergebildet werden, dass der Boden des Ringkanals etwa auf der Höhe des Bodens der topfartigen Vertiefung liegt. Im einzelnen ist es ferner bevorzugt, dass der Ringkanal den den freien Ringraum aussen begrenzenden Vorsprung aufnimmt.

Eine Weiterbildung nach der Erfindung ist dadurch gekennzeichnet, dass der den freien Ringraum begrenzende Vorsprung und die Aussenwandung je eine Aussparung für den Durchtritt der Zunge an der Filtermembran aufweisen. Hierdurch wird eine Verformung der Filtermembran in dem an die Zunge anschliessenden Bereich vermieden.

Im folgenden wird die Erfindung anhand einer in den Zeichnungen beispielhaft veranschaulichten Ausführungsform näher erläutert. Es zeigt:
**FIG. 1** eine perspektivische Ansicht des erfindungsgemässen Filters in auseinandergezogener Darstellung;
**FIG. 2** eine Seitenansicht des geschlossenen Filters gemäss Figur 1;
**FIG. 3** eine Unteransicht des Filters gemäss den Fig. 1 und 2;
**FIG. 4** eine Draufsicht des Filters gemäss den Fig. 1 und 2;
**FIG. 5** eine Draufsicht auf das Unterteil des Filters gemäss Fig. 1 und 2;
**FIG. 6** eine Schnittansicht längs der Linie A-A in Fig. 2;
**FIG. 7** eine Schnittansicht des Bereiches des Filters gemäss Fig. 1 und 2, in welchem die an der Filtermembran vorgesehene Zunge nach aussen aus dem Gehäuse vorsteht, und
**FIG. 8** eine Seitenansicht bzw. Draufsicht des Filters gemäss den Fig. 1 bis 7 in natürlicher Grösse.

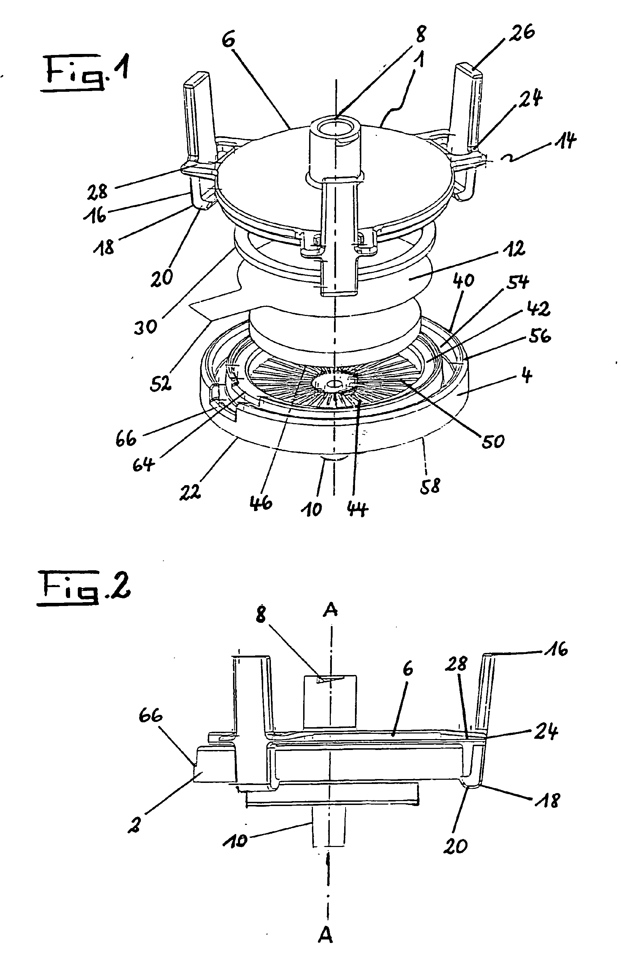

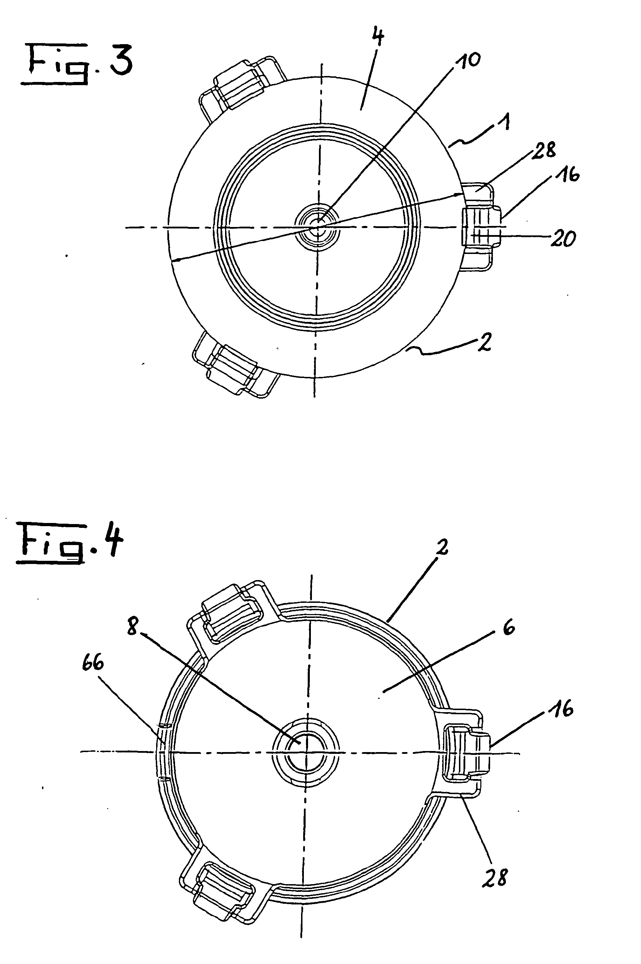

Wie in den Zeichnungen, insbesondere Fig. 1 bis 4, gezeigt, weist der Filter 1 nach der Erfindung ein zweiteiliges Gehäuse 2 aus Kunststoff auf, welches aus einem Unterteil 4 und einem Deckel 6 besteht. Der Deckel 6 hat einen zentralen Einlass 8, welcher beim Ausführungsbeispiel als weiblicher Luer-Lock-Anschluss ausgebildet ist. Der Unterteil 4 ist mit einem Auslass 10 versehen, welcher ebenfalls entsprechend dem Einsatzgebiet des Filters 1 für medizinische und Laborzwecke als männlicher Luer-Lock-Anschluss ausgebildet ist.

Zwischen Deckel und Unterteil ist eine auswechselbare Filtermembrane 12 eingeklemmt, wobei eine allgemein mit 14 versehene Verbindungseinrichtung vorgesehen ist, um den Deckel 6 mit dem Unterteil 4 mediendicht zu verbinden.

Erfindungsgemäss ist die allgemein mit 14 bezeichnete Verbindungseinrichtung als um den Umfang des Deckels in Abständen angeordnete Federhebel 16 ausgebildet. Die freien Unterenden 18 der Federhebel 16 sind mit hakenförmigen, nach innen gerichteten Vorsprüngen 20 versehen, welche unter Erzeugung einer Anpresskraft zwischen dem Deckel 6 und dem Unterteil 4 formschlüssig mit dem Unterteil verbindbar sind, indem in der in den Fig. 2 bis 4 dargestellten geschlossenen Stellung des Gehäuses 2 die hakenförmigen Vorsprünge 20 den Unterrand 22 des Unterteils 4 lösbar übergreifen. Die Anordnung ist hierbei derart getroffen, dass die Federhebel 16 in Schliessrichtung federvorgespannt sind.

Um das Gehäuse 2 des Filters 1 zum Wechseln der Filtermembran 12 öffnen zu können, sind die Federhebel 16 oberhalb ihres Drehpunkts 24 mit nach oben vorstehenden Betätigungslaschen 26 versehen. Durch Zusammendrücken der Betätigungslaschen 26 gegen die nach innen gerichtete Vorspannung der Federhebel 16 lässt sich der Deckel 6 leicht von dem Unterteil trennen.

Bei der veranschaulichten Ausführungsform nach der Erfindung sind die Federhebel 16 einstückig mit dem aus Kunststoff bestehenden Deckel ausgebildet, indem die Federhebel 16 an seitlich von dem Deckel 6 vorstehenden bogenförmigen Laschen 28 angespritzt sind. Die Laschen 28 bilden hierbei den Drehpunkt 24 der Federhebel 16 und erzeugen gleichzeitig die auf die Mittelachse des Gehäuses 1 zu gerichtete Federvorspannung der Federhebel nach innen.

Bei dem veranschaulichten Ausführungsbeispiel sind drei derartige Federhebel 16 um den Umfang des Deckels 6 in gleichen Abständen verteilt vorgesehen, da durch diese Form der Dreipunktbefestigung eine sichere Anlage des Deckels 6 an dem Unterteil 4 gewährleistet ist.

Es ist offensichtlich, dass die Anordnung auch umgekehrt erfolgen kann, indem die Federhebel an das Unterteil 4 angespritzt sind und den Oberrand des Deckels 6 übergreifen. Aus praktischen Gründen, d.h. zur Erleichterung der Bedienung und Trennung von Deckel 6 und Unterteil 4, wurde jedoch die veranschaulichte Anordnung mit den Federhebeln 16 am Deckel 6 gewählt.

Wie aus den Fig. 1 und 6 ersichtlich, ist zwischen dem Deckel 6 und dem Unterteil 4 eine zusammendrückbare Ringdichtung 30 vorgesehen, welche in einem ringförmigen Kanal in der Unterseite 34 des Deckels 6 angeordnet bzw. befestigt ist.

Der Kanal ist auf seiner radial ausserhalb der Ringdichtung 30 liegenden Seite durch einen ringförmigen Vorsprung begrenzt, welcher zwischen der Ringdichtung 30 und dem Vorsprung einen freien Ringraum 36 definiert. Auf der radial innenliegenden Seite ist der Kanal durch einen Ringwulst 37 begrenzt, an welchem die Ringdichtung 30 anliegt und welcher, wie aus Fig. 6 ersichtlich, teilweise auf der Filtermembran 12 aufliegt.

In den freien Ringraum 36 greift ein ringförmiger Vorsprung auf der Oberseite 40 des Unterteils 4 ein, welcher gleichzeitig eine Auflagefläche 42 als Gegenfläche zur Ringdichtung 30 umgibt.

Wie aus den Fig. 1 und 6 ersichtlich, ist die Filtermembran in ihrem Durchmesser dem Innendurchmesser des ringförmigen Vorsprungs angepasst, so dass bei geschlossenem Gehäuse 2 der Randbereich der Filtermembrane 12 zwischen der Ringdichtung 30 und der Auflagefläche 42 eingeklemmt ist.

Wie ferner aus den Fig. 1, 5 und 6 ersichtlich, weist der Unterteil 4 eine topfartige Vertiefung 44 innerhalb der Auflagefläche 42 auf, in welcher ein scheibenförmiger Stützkörper 46 bevorzugt aus porösem Werkstoff für die Filtermembran 12 angeordnet ist. Bei geschlossenem Gehäuse 2 ist daher die Filtermembran 12 einerseits an ihrem Rand zwischen der Ringdichtung und der Auflagefläche 42 eingeklemmt und andererseits in ihrem Mittelbereich durch den Stützkörper 46 abgestützt.

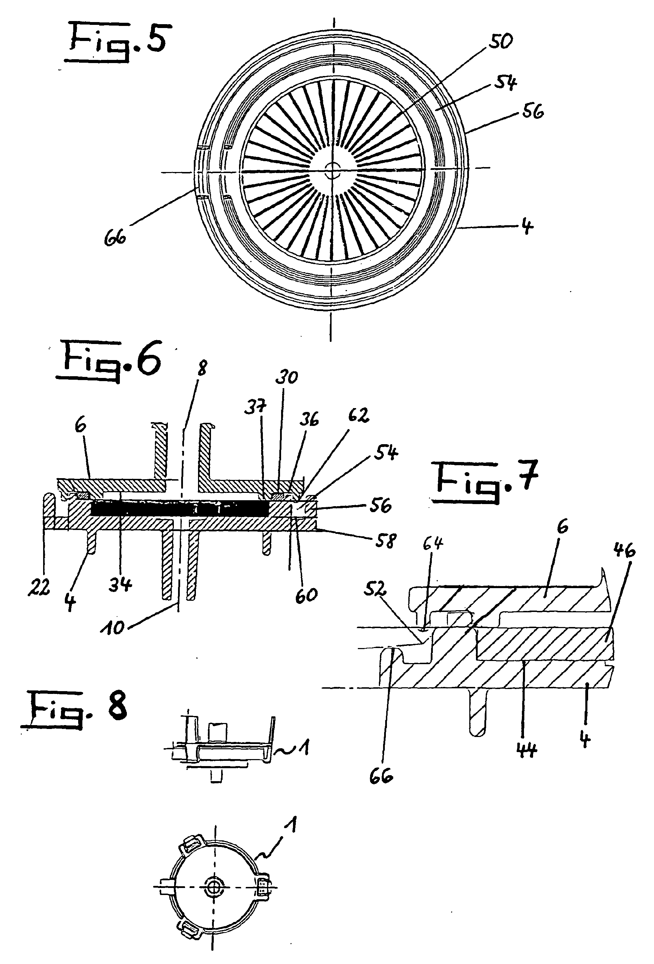

Aus den Fig. 1 und 5 ist ersichtlich, dass der Boden der topfartigen Vertiefung 44 in dem Unterteil 4 bevorzugt mit Stützrippen 50 für den Stützkörper 46 versehen ist, welche sternförmig den zentral angeordneten Auslass 10 umgeben.

Bei dem veranschaulichten bevorzugten Ausführungsbeispiel ist die Filtermembran 12, wie insbesondere aus den Fig. 1 und 7 ersichtlich, mit einer Zunge 52 versehen, welche seitlich aus dem geschlossenen Gehäuse vorsteht. Die Zunge 52 dient der verbesserten Handhabung der Filtermembran 12 bei deren Wechsel und ermöglicht gleichzeitig eine Identifikation der zur Zeit im Gehäuse befindlichen Membran bzw. der gerade durchgeführten Analyse oder Arbeit.

Aus den Fig. 1, 5 und 6 ist zu sehen, dass radial ausserhalb des ringförmigen Vorsprungs an dem Unterteil 4 ein weiterer Ringkanal 54 vorgesehen ist, dessen Aussenwandung 56 an der äusseren Unterkante 58 von den hakenförmigen Vorsprüngen 20 der Federhebel 16 übergriffen wird. Der Boden 60 des Ringkanals 54 liegt etwa auf der Höhe des Bodens der topfartigen Vertiefung 44.

Wie die Schnittdarstellung gemäss Fig. 6 zeigt, nimmt der Ringkanal 54 den den freien Ringraum 36 am Deckel 6 aussen begrenzenden Vorsprung 62 auf.

Wie aus der vergrösserten Detailansicht gemäss Fig. 7 und auch aus der Draufsicht auf den Unterteil 4 ersichtlich, weisen der den freien Ringraum 36 begrenzende Vorsprung 62 und die Aussenwandung 56 je eine Aussparung 64 bzw. 66 auf, welche die nach aussen aus dem Gehäuse 2 vorstehende Zunge 52 der Filtermembran 12 aufnehmen.

Sämtliche aus der Beschreibung, den Ansprüchen und Zeichnungen hervorgehenden Merkmale und Vorteile der Erfindung, einschliesslich konstruktiver Einzelheiten und räumlicher Anordnungen, können sowohl für sich als auch in beliebiger Kombination erfindungswesentlich sein.

### BEZUGSZEICHENLISTE

- 1 =: Filter
- 2 =: Gehäuse
- 4 =: Unterteil
- 6 =: Deckel
- 8 =: Einlass
- 10 =: Auslass
- 12 =: Filtermembrane
- 14 =: Verbindungseinrichtung
- 16 =: Federhebel
- 18 =: freies Unterende v. 16
- 20 =: hakenförmiger Vorsprung
- 22 =: Unterrand v. 4
- 24 =: Drehpunkt v. 16
- 26 =: Betätigungslasche
- 28 =: Lasche
- 30 =: Ringdichtung
- 34 =: Unterseite v. 6
- 36 =: Ringraum
- 37 =: Ringwulst
- 40 =: Oberseite v. 4
- 42 =: Auflagefläche
- 44 =: Vertiefung
- 46 =: Stützkörper
- 50 =: Stützrippen
- 52 =: Zunge
- 54 =: Ringkanal
- 56 =: Aussenwandung
- 58 =: Unterkante
- 60 =: Boden v. 54
- 62 =: Vorsprung um 36
- 64 =: Aussparung
- 66 =: Aussparung

## Patentansprüche

1. Filter (1) für medizinische und Laborzwecke, insbesondere für Blutanalysen und dergleichen, mit einem zweiteiligen, aus einem Unterteil (4) und einem Deckel (6) bestehenden Gehäuse (2) aus Kunststoff, wobei der Deckel (6) einen Einlass (8) und der Unterteil (4) einen Auslass (10) aufweist, und wobei zwischen Deckel (6) und Unterteil (4) eine auswechselbare Filtermembran (12) eingeklemmt ist und eine den Deckel mit dem Unterteil mediendicht verbindende Verbindungseinrichtung (14) vorgesehen ist, wobei die Verbindungseinrichtung (14) als um den Umfang des Deckels (6) in Abständen angeordnete Federhebel (16) ausgebildet ist, deren freie Unterenden (18) mit hakenförmigen Vorsprüngen (20) unter Erzeugung einer Anpresskraft zwischen Deckel (6) und Unterteil (4) formschlüssig mit dem Unterteil (4) verbindbar sind, **dadurch gekennzeichnet, dass** die hakenförmigen Vorsprünge (20) den Unterrand (22) des Unterteils (4) lösbar übergreifen, und dass die Federhebel (16) oberhalb ihres Drehpunkts (24) nach oben vorstehende Betätigungslaschen (26) aufweisen.

2. Filter nach Anspruch 1, **dadurch gekennzeichnet, dass** die Federhebel (16) einstückig mit dem aus Kunststoff bestehenden Deckel (6) ausgebildet sind, und dass die Federhebel (16) an seitlich von dem Deckel (6) vorstehenden bogenförmigen Laschen (28) angeformt sind, welche den Drehpunkt (24) der Federhebel (16) bilden.

3. Filter nach einem der vorstehenden Ansprüche, **dadurch gekennzeichnet, dass** drei Federhebel (16) um den Umfang des Deckels (6) in gleichen Abständen verteilt vorgesehen sind.

4. Filter nach einem der vorstehenden Ansprüche, **dadurch gekennzeichnet, dass** zwischen dem Deckel (6) und dem Unterteil (4) eine zusammendrückbare Ringdichtung (30) vorgesehen ist, dass die Ringdichtung (30) in einem ringförmigen Kanal (32) in der Unterseite (34) des Deckels (6) angeordnet bzw. befestigt ist, und dass der Kanal (32) radial außerhalb der Ringdichtung (30) einen freien Ringraum (36) aufweist, in welchen ein ringförmiger Vorsprung (38) auf der Oberseite (40) des Unterteils (34) eingreift, welcher eine Auflagefläche (42) der Ringdichtung (30) umgibt.

5. Filter nach Anspruch 4, **dadurch gekennzeichnet, dass** die Filtermembran (12) in ihrem Durchmesser dem Innendurchmesser des ringförmigen Vorsprungs (38) entspricht, derart, dass bei geschlossenem Gehäuse (2) der Randbereich der Filtermembran (12) zwischen Ringdichtung (30) und der Auflagefläche (42) eingeklemmt ist, dass in einer topfartigen Vertiefung (44) des Unterteils (4) innerhalb der Auflagefläche (42) ein scheibenförmiger Stützkörper (46) für die Filtermembran (12) vorgesehen ist, und dass der Boden (18) der Vertiefung (44) mit sternförmig, den zentralen Auslass (10) umgebenden Stützrippen (50) versehen ist.

6. Filter nach Anspruch 5, **dadurch gekennzeichnet, dass** der Stützkörper (46) aus einem porösen Werkstoff besteht.

7. Filter nach einem der vorstehenden Ansprüche, **dadurch gekennzeichnet, dass** die Filtermembran (12) mit einer seitlichen, aus dem geschlossenen Gehäuse (2) vorstehenden Zunge (52) versehen ist.

8. Filter nach einem der vorstehenden Ansprüche, **dadurch gekennzeichnet, dass** radial ausserhalb des ringförmigen Vorsprungs (38) an dem Unterteil (4) ein weiterer Ringkanal (54) vorgesehen ist, dessen Aussenwandung (56) an der äusseren Unterkante (58) von den hakenförmigen Vorsprüngen (20) übergriffen wird, und dass der Boden (60) des Ringkanals (54) etwa auf der Höhe des Bodens (48) der Vertiefung (44) liegt.

9. Filter nach Anspruch 8, **dadurch gekennzeichnet, dass** der Ringkanal (54) den, den freien Ringraum (36) aussen begrenzenden Vorsprung (62) aufnimmt, und dass der den freien Ringraum (36) begrenzende Vorsprung (62) und die Aussenwandung (56) je eine Aussparung (64, 66) für den Durchtritt der Zunge (52) aufweisen.

## Claims

1. A filter (1) for medical and laboratory use, especially for blood analysis and the like, having a two-part plastic housing (2) consisting of a bottom part (4) and a cover (6), wherein the cover (6) is having an inlet (8) and the bottom part (4) is having an outlet (10) and, wherein an exchangeable filter diaphragm (12) is clamped between the cover (6) and the bottom part (4) with a connection means (14) being provided to connect the cover (6) with the bottom part (4) in a fluid-tight manner, wherein the connection means (14) is provided as a number of spring levers (16) distributed around the circumference of the cover (6), the free bottom ends (18) of which by the means of hook-shaped projections (20) positively are connectable with the bottom part (4) creating a contact pressure between the cover (6) and the bottom part (4), **characterised in that** the hook-shaped projections (20) releasably are overlapping the bottom rim (22) of the bottom part (4) and, that the spring levers (16) are having upwardly projecting actuation flaps (26) above their pivot point (24).

2. Filter according to claim 1, **characterised in that** the spring levers (16) are performed unitary with the cover (6) consisting of plastic, and **in that** the spring levers (16) are moulded onto bow-shaped flaps (28) laterally projecting from the cover (6), which are forming the pivot point (24) of the spring levers (16).

3. Filter according to any of the preceding claims, **characterised in that** there are provided three spring levers (16), which are distributed around the circumference of the cover (6) at equal distances.

4. Filter according to any of the preceding claims, **characterised in that** a compressible annular seal (30) is provided between the cover (6) and the bottom part (4), that the annular seal (30) is positioned or fixed, respectively, in an annular channel (32) in the bottom side (34) of the cover (6) and, **in that** the channel (32) radially outward of the annular seal (30) is having a free annular space (36) into which an annular projection (38) on the top side (40) of the bottom part (34) is engaging, which is surrounding a contact surface (42) of the annular seal (30).

5. Filter according to claim 4, **characterised in that** the filter diaphragm (12) is having a diameter corresponding to the interior diameter of the annular projection (38) such that with the housing being closed the circumferential rim of the filter diaphragm (12) is clamped between the annular seal (30) and the contact surface (42), that a disk-shaped supporting body (46) for the filter diaphragm (12) is provided in a pot-shaped recess (44) of the bottom part (4) radially inward of the contact surface (42) and, that the bottom (18) of the recess (44) is provided with supporting ribs (50) surrounding the central outlet (10) in a star-shaped pattern.

6. Filter according to claim 5, **characterised in that** the supporting body (46) is consisting of a porous material.

7. Filter according to any of the preceding claims, **characterised in that** the filter diaphragm (12) is having a lateral tongue (52) projecting from the closed housing (2).

8. Filter according to any of the preceding claims, **characterised in that** radially outwardly of the ring-shaped projection (38) on the bottom part there is provided a further annular channel (54), the exterior wall (56) of which is overlapped by the hook-shaped projections (20) and, that the bottom (60) of the annular channel (54) is positioned generally at the height of the bottom (48) of the recess (44).

9. Filter according to claim 8, **characterised in that** the annular channel (54) is receiving the projection (62) surrounding the free annular space (36) and, that the projection (62) limiting the free annular space (36) and the exterior wall (56) each are having a recess (64, 66) for the passage of the tongue (52).

## Revendications

1. Filtre (1) à des fins médicales et de laboratoire, en particulier pour des analyses sanguines et analogues, avec un boîtier (2) en matière plastique en deux parties, consistant en une partie inférieure (4) et en un couvercle (6), où le couvercle (6) comporte une entrée (8) et la partie inférieure (4) comporte une sortie (10), et où une membrane filtrante (12) remplaçable est serrée entre le couvercle (6) et la partie inférieure (4) et un dispositif de liaison (14) reliant le couvercle et la partie inférieure de manière étanche aux milieux est prévu, où le dispositif de liaison (14) est agencé sous forme de leviers à ressort (16) disposés à intervalles autour de la périphérie du couvercle (6), dont les extrémités inférieures libres (18) peuvent être reliées par assemblage de forme avec la partie inférieure (4) avec des protubérances en forme de crochet (20) en produisant une force de pression entre le couvercle (6) et la partie inférieure (4), **caractérisé en ce que** les protubérances en forme de crochet (20) passent sur le bord inférieur (22) de la partie inférieure (4) de manière amovible, et **en ce que** les leviers à ressort (16) comportent au-dessus de leur point d'appui (24) des languettes de manoeuvre (26) qui font saillie vers le haut.

2. Filtre selon la revendication 1, **caractérisé en ce que** les leviers à ressort (16) sont agencés d'une pièce avec le couvercle (6) consistant en matière plastique et **en ce que** les leviers à ressort (16) sont formés au niveau de languettes (28) en forme d'arc qui font saillie latéralement du couvercle (6), et qui forment le point d'appui (24) des leviers à ressort (16).

3. Filtre selon l'une des revendications précédentes, **caractérisé en ce qu'**il est prévu trois leviers à ressort (16) répartis à intervalles réguliers autour de la périphérie du couvercle (6).

4. Filtre selon l'une des revendications précédentes, **caractérisé en ce qu'**il est prévu un joint d'étanchéité annulaire (30) qui peut être compressé entre le couvercle (6) et la partie inférieure (4), **en ce que** le joint d'étanchéité annulaire (30) est disposé ou fixé dans un canal annulaire (32) dans la partie inférieure (34) du couvercle (6), et **en ce que** le canal (32) comporte radialement à l'extérieur du joint d'étanchéité annulaire (30) un espace annulaire libre (36) dans lequel pénètre une protubérance annulaire (38) sur le côté supérieur (40) de la partie inférieure (34), qui entoure une surface d'appui (42) du joint d'étanchéité annulaire (30).

5. Filtre selon la revendication 4, **caractérisé en ce que** la membrane filtrante (12) correspond concernant son diamètre au diamètre interne de la protubérance annulaire (38) de telle manière que, lorsque le boîtier (2) est fermé, le domaine de bordure de la membrane filtrante (12) est serré entre le joint d'étanchéité annulaire (30) et la surface d'appui (42), **en ce qu'**il est prévu un corps de support (46) en forme de disque pour la membrane filtrante (12) dans un évidement en forme de pot (44) de la partie inférieure (4) à l'intérieur de la surface d'appui (42), et **en ce que** le fond (18) de l'évidement (44) est muni de nervures de support (50) en forme d'étoile qui entourent la sortie centrale (10).

6. Filtre selon la revendication 5, **caractérisé en ce que** le corps de support (46) consiste en un matériau poreux.

7. Filtre selon l'une des revendications précédentes, **caractérisé en ce que** la membrane filtrante (12) est munie d'une languette (52) latérale qui fait saillie du boîtier (2) fermé.

8. Filtre selon l'une des revendications précédentes, **caractérisé en ce qu'**il est prévu radialement à l'extérieur de la protubérance annulaire (38) sur la partie inférieure (4) un autre canal annulaire (54) sur la paroi extérieure (56) duquel passent les protubérances en forme de crochet (20) au niveau du bord inférieur externe (58), et **en ce que** le fond (60) du canal annulaire (54) est situé sensiblement à la hauteur du fond (48) de l'évidement (44).

9. Filtre selon la revendication 8 **caractérisé en ce que** le canal annulaire (54) reçoit la protubérance (62) limitant extérieurement l'espace annulaire libre (36) et **en ce que** la protubérance (62) limitant l'espace annulaire libre (36) et la paroi extérieure (56) comportent chacune un évidement (64, 66) pour le passage de la languette (52).
